# EUROPÄISCHE PATENTSCHRIFT

(11) **EP 2 745 768 B1**
(45) Veröffentlichungstag und Bekanntmachung des Hinweises auf die Patenterteilung: **16.09.2015**
(21) Anmeldenummer: 13196240.9
(22) Anmeldetag: 09.12.2013
(51) Int. Cl.: A61B 5/00

(54) **Medizinischer Indikator für den diabetischen Fuß**
Medical indicator for the diabetic foot
Indicateur médical pour le pied diabétique

(30) Priorität: 18.12.2012 DE 102012112458
(43) Veröffentlichungstag der Anmeldung: 25.06.2014
(73) Patentinhaber: Cremerius, Alois, 41379 Brüggen (DE); Zick, Reinhard, 40809 Lingen (DE)
(72) Erfinder: Cremerius, Alois, 41379 Brüggen (DE); Zick, Reinhard, 40809 Lingen (DE)
(74) Vertreter: Frese Patent

(56) Entgegenhaltungen:
- EP-B1- 1 408 834
- US-A- 6 113 551
- US-B1- 6 200 272

## Beschreibung

Die Erfindung betrifft eine Vorrichtung zur medizinischen Früherkennung eines diabetischen Fußes, wobei mit Hilfe der Vorrichtung eine Druckempfindlichkeit des Fußes geprüft wird, wobei die Vorrichtung einen Klebestreifen umfasst, an dem ein druckfester Körper als erster Reizgeber befestigt ist.

Der Klebestreifen ist z.B. ein Pflaster und der Körper z.B. kugel-, ellipsoid- oder tropfenförmig.

Der diabetische Fuß (DF) ist eine Folge der Zuckerkrankheit (Diabetes mellitus) und ist auf eine mangelnde Durchblutung der Beine, insbesondere die arterielle Verschlusskrankheit (AVK), und auf eine Nervenstörung (Polyneuropathie) zurückzuführen. Wenn dies nicht rechtzeitig erkannt wird, muss gegebenenfalls eine entsprechende Amputation durchgeführt werden, was mit Schmerzen und Behinderungen des Patienten einerseits und finanzieller Belastung der Krankenkassen andererseits verbunden ist. Um Amputationen - zur Zeit in Deutschland etwa 40.000 pro Jahr - so weit wie möglich zu vermeiden, ist eine Früherkennung des diabetischen Fußes unumgänglich, damit den Krankheitsfolgen durch geeignete Therapien so früh wie möglich entgegengesteuert werden kann.

Zur Behandlung der Zuckerkrankheit wird in erster Linie der Blutzucker- Wert des Patienten mittels Medikamenten möglichst nahe an einen Normwert eingestellt. Dies kann aber nur dann optimal erfolgen, wenn der Patient durch regelmäßige Bewegung auch Zucker verbraucht; hierfür sind gesunde Füße äußerst wichtig. Ist die Bewegung und damit die optimale Blutzucker- Einstellung nicht möglich, sind weitere Folgeschäden der Krankheit an Nerven, Augen, Herz, Gefäßen, Nieren und im Magen-Darm-Trakt zu befürchten. Daher ist die Früherkennung des Diabetischen Fußes sowohl für den behandelnden Arzt als auch für den Patienten besonders wichtig.

Aus der DE 100 18 790 A1 ist ein Indikatorpflaster zur Erkennung des Diabetischen Fußes bekannt, das eine zu trockene Haut erkennt. Dieses Pflaster umfasst ein mit Kobalt-(II)-Chlorid imprägniertes Teil, das in trockenem Zustand blau ist. Zum Gebrauch wird das Pflaster auf die Fußsohle eines Patienten geklebt. Bei normal feuchtem Fuß wird das imprägnierte Teil nach einer bestimmten Zeit rot; fehlt dieser Farbumschlag, bleibt also das Teil blau, deutet dies auf ungewöhnliche Trockenheit des Fußes und somit auf den diabetischen Fuß hin.

Die WO 97/31599 A beschreibt ein Indikatorpflaster, das über eine chemische Reaktion den Glucosegehalt des Blutes anzeigt. Hiermit kann ein Nachweis der Zuckerkrankheit geführt werden.

In der EP 430 608 A1 ist ein Indikatorpflaster zur Diagnose der Zuckerkrankheit offenbart, das mit Hilfe eines temperaturempfindlichen Flüssigkristall- Bandes Informationen als Folge von thermisch-chemischen Reaktionen liefert.

Die EP 1 408 834 B1, von der die Erfindung ausgeht, betrifft ein medizinisches Indikatorpflaster mit einer druckfesten Kugel, mit dessen Hilfe die Druckempfindlichkeit der Fußsohle als Reizgeber (Indikator) für den Diabetischen Fuß geprüft wird.

Bei der Untersuchung der Druckempfindlichkeit des Fußes eines Zuckerkranken ist es zumindest wünschenswert, unterschiedlich starke Druckreizungen zu prüfen. Für diese Prüfungen sind bereits unterschiedliche Vorrichtungen bekannt, die jeweils separat gefertigt sind. Zu diesen Vorrichtungen gehören ein Monofilament, das an einem Halter befestigt ist, das oben genannte Indikatorpflaster mit einer Kugel sowie eine Scheibe mit unterschiedlichen Reizgebern. Diese Reizgeber sind am äußeren Umfang der Scheibe z.B. als spitze und runde radiale Grate ausgebildet.

Aus hygienischen Gründen sollen die Vorrichtungen nur einmal verwendet werden.

Der Nachteil der bekannten Vorrichtungen zur Untersuchung der Druckempfindlichkeit des diabetischen Fußes besteht in der Vielzahl der unterschiedlichen Reizgeber (Indikatoren), was mit entsprechenden Herstell- Verpackungs- und Entsorgungskosten sowie Kosten der Verteilung und Lagerung verbunden ist.

Es ist daher die Aufgabe der Erfindung, eine Vorrichtung zur Untersuchung der Druckempfindlichkeit zu schaffen, in der mehrere Reizgeber für eine gestuft zu prüfende Reizempfindlichkeit des diabetischen Fußes kombiniert sind, wobei insgesamt Kosten gespart werden.

Die Aufgabe ist dadurch gelöst, dass als zweiter Reizgeber ein Monofilament mit einer definierten Steifigkeit angeordnet ist, das mit einem ersten Ende in einer Bohrung des Körpers geführt ist und mit einem zweiten Ende über einen äußeren Rand des Klebestreifens für eine vorbestimmte Länge herausragt.

In einer alternativen Ausführung der Erfindung besteht die Lösung der Aufgabe darin, dass als zweiter Reizgeber ein Monofilament mit einer definierten Steifigkeit angeordnet ist, das einteilig an den Körper angeformt ist und mit einem zweiten Ende über einen äußeren Rand des Klebestreifens für eine vorbestimmte Länge herausragt.

Hierbei ist bevorzugt zwischen dem Körper und dem Monofilament eine Sollbruchstelle ausgebildet.

Bei beiden Lösungen ist ein Endbereich des Monofilaments an dem Klebestreifen festgelegt, so dass dieser einen Halter des Monofilaments für die Durchführung der Prüfung der Druckempfindlichkeit einer Fußsohle eines Patienten bildet. Weil das erste Ende des Monofilaments in der Bohrung des Körpers geführt beziehungsweise an diesem angeformt ist, ist das Monofilament bei Ausübung von Druck auf sein zweites Ende zusätzlich gegen ein Verrutschen in/an dem Pflaster gesichert, so dass diese Prüfung, die einen relativ geringen Reiz ausübt, einfach und sicher wie gewohnt durchgeführt werden kann.

Nach dieser Prüfung wird das Monofilament von dem Klebestreifen ab- und aus dem Körper herausgezogen beziehungsweise von diesem an der Sollbruchstelle abgebrochen, so dass dann die weitere Prüfung mit größerem Reiz mit Hilfe des Klebestreifens und dem Körper durchgeführt werden kann. Hierfür wird eine Schutzfolie von dem Klebestreifen abgezogen, die eine Schicht aus Klebstoff schützt, das Monofilament wird entfernt und der Klebestreifen auf die Fußsohle des Patienten geklebt. Der Patient soll dann mit dem aufgeklebten Klebestreifen gehen und die durch den Körper bewirkte Schmerzempfindung bewerten.

Das Monofilament ist ein Kunststofffaden mit definierter freier Länge und Steifigkeit und sollte einen bestimmten Durchmesser aufweisen. Hiermit lässt sich ein wesentlich feinfühligeres Druckempfinden feststellen als mit dem Klebestreifen mit der Körper.

Ein separater Halter und eine Schutzhülle für das Monofilament werden eingespart, so dass die erfindungsgemäße Vorrichtung insgesamt preiswerter zu fertigen ist als die entsprechenden separaten Vorrichtungen. Es muss nur eine Vorrichtung für die Ausübung der unterschiedlichen Reize bestellt werden, was den Verwaltungsaufwand vermindert.

Die Vorrichtung kann sowohl von einem Arzt als auch vom Patienten selbst verwendet werden, wobei ein abgestuftes Druckempfinden feststellbar ist. Dieses abgestufte Druckempfinden zeigt möglicherweise auch eine psychische Wirkung, wenn der Patient bei der Anwendung des Monofilaments keinen Druck spürt, wohl aber bei der Verwendung des Klebestreifens mit dem Körper. Bei der Selbstanwendung kann der Patient mit geringem Aufwand seine Druck- und Schmerzempfindlichkeit überprüfen, so dass bei einer beginnenden Verschlechterung sofort geeignete Gegenmaßnahmen ergriffen werden können.

Die Vorrichtung ist als Einwegartikel vorgesehen. Daher sind hygienische Standards sicher einzuhalten. Eine personalintensive Reinigung/Sterilisierung entfällt.

Die Unteransprüche betreffen die vorteilhafte Ausgestaltung der Erfindung.

In einer Ausgestaltung der Erfindung umfasst die Vorrichtung eine steife Scheibe, auf der der Klebestreifen zusammen mit dem Körper und dem Monofilament befestigt ist und an deren Außenumfang mindestens ein weiterer Reizgeber geformt ist. Hierdurch kann die Reizempfindlichkeit des Fußes in weiteren Abstufungen geprüft werden. Die weiteren Reizgeber sind nach vorgegebenen Standards geformt.

In einer weiteren Ausgestaltung ist die Scheibe aus Pappe gefertigt. Pappe ist preiswert in der erforderlichen Stabilität zu erhalten und einfach zu recyceln.

In einer weiteren Ausgestaltung ist der Körper als Kugel mit einem Durchmesser von 1 mm bis 10 mm ausgebildet. Dies gewährleistet einen ausreichenden Druck auf die Fußsohle.

In einer weiteren Ausgestaltung zeigt das Monofilament ab einer Kraft von 10 mN eine sichtbare Durchbiegung. Dies entspricht den standardisierten Vorgaben.

Die Erfindung wird anhand der beigefügten schematischen Zeichnung weiter erläutert. Es zeigen
Figur 1 eine Draufsicht einer Vorrichtung zur medizinischen Früherkennung eines diabetischen Fußes mit einer Scheibe,
Figur 2 einen Längsschnitt durch die Vorrichtung gemäß Figur 1 und
Figur 3 eine Draufsicht einer alternativen Ausgestaltung einer Kugel mit einem Monofilament.

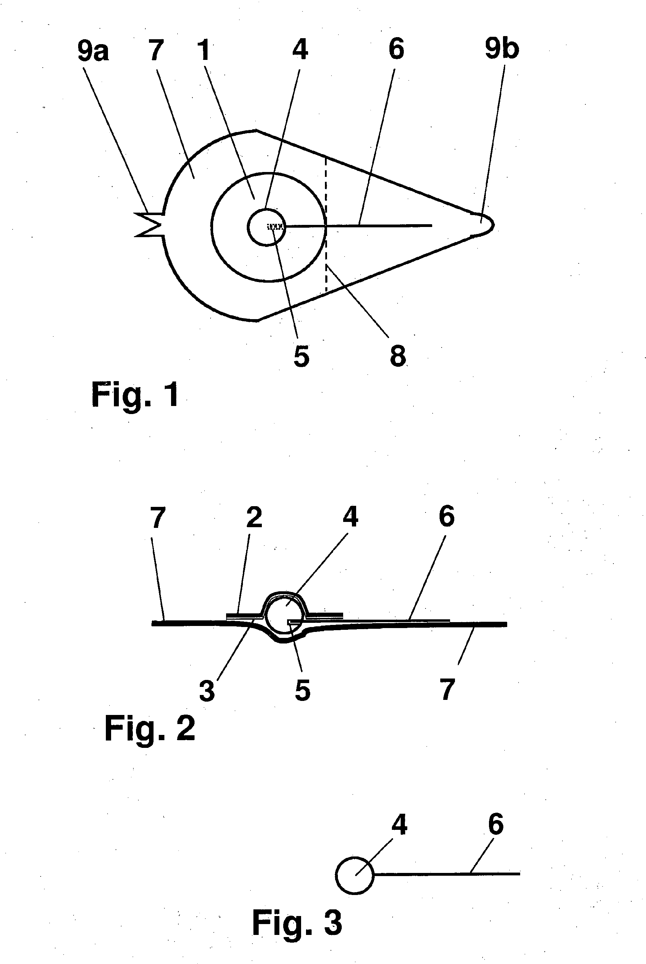

Wie aus den Figuren 1 und 2 ersichtlich umfasst eine Vorrichtung zur medizinischen Früherkennung eines diabetischen Fußes in einer ersten Ausführung der Erfindung einen Klebestreifen, hier ein Pflaster 1, und eine steife Scheibe 7.

Das Pflaster 1 umfasst einen Träger 2, der einseitig mit einem Klebstoff 3 beschichtet ist. Der Träger 2 ist z.B. aus einem Gewebe gefertigt und weist vorzugsweise eine runde Hauptfläche auf. In etwa zentrisch auf dem Träger 2 ist auf dem Klebstoff 3 ein druckfester Körper, hier eine Kugel 4, als erster Reizgeber angeordnet und durch den Klebstoff 3 gehalten. Die Kugel 4 ist aus einem harten Material wie Kunststoff oder Metall gefertigt und weist eine Sackbohrung 5 auf, die nicht radial in die Kugel 4 eingelassen ist. In der Sackbohrung 5 ist ein erstes Ende eines Monofilaments 6 geführt, das sich von der Kugel 4 weitgehend geradlinig für eine vorbestimmte Länge über einen Rand des Trägers 2 hinaus erstreckt. Ein Durchmesser der Kugel 4 beträgt 1 mm bis 10 mm, insbesondere 2 mm bis 5 mm. Als besonders günstig hat sich ein Durchmesser von 3,5 mm erwiesen. Ein Durchmesser der Sackbohrung 5 entspricht dem des Monofilaments 6 zuzüglich eines geringen Spiels.

Das Monofilament 6 als zweiter Reizgeber ist aus Kunststoff so gefertigt, dass es sich bei der vorbestimmten Länge ab einer Kraft von 10 mN sichtbar durchbiegt. Hierbei weist das Monofilament 6 vorzugsweise eine festgelegte Querschnittsfläche auf, damit bei der Durchführung einer Prüfung einer Druckempfindlichkeit einer Fußsohle ein definierter Druck ausgeübt wird und so vergleichbare Ergebnisse erzielt werden. Im Bereich des Trägers 2 ist das Monofilament 6 außer durch die Kugel 4 auch durch den Klebstoff 3 lösbar gehalten, so dass es während der Prüfung gegen Verrutschen gesichert ist.

Die Scheibe 7 weist eine deutlich größere Hauptfläche auf als das Pflaster 1, ist z.B. aus Pappe oder Kunststoff gefertigt und zumindest einseitig mit einer Antihaftschicht beschichtet. Die Hauptfläche der Scheibe 7 ist hier in etwa tropfenförmig ausgebildet. Am Außenumfang der Scheibe 7 sind unterschiedliche weitere Reizgeber angeordnet wie z.B. paarweise angeordnete spitze Grate 9a mit definiertem Abstand und ein Oval 9b. Bevorzugt sind mehrere Grate 9a mit unterschiedlichen Abständen als Teil der weiteren Reizgeber paarweise angeordnet.

Der Träger 2 einschließlich der Kugel 4 und dem Monofilament 6 ist mit der mit dem Klebstoff 3 beschichteten Seite auf der Antihaftschicht der Scheibe 7 leicht lösbar befestigt. Hierbei weist ein zweites Ende des Monofilaments 6 in einer Längsachse der Scheibe 7 zu einer Spitze des Tropfens, hier zu dem Oval 9b. Die Scheibe 7 ist senkrecht zu dem Monofilament 6 und tangential zu dem Träger 2 mit einer Perforation 8 versehen.

An der Vorrichtung können weitere Indikatoren angeordnet sein.

Die gesamte Vorrichtung kann von einer Schutzhülle umgeben sein.

Eine zweite Ausführung der Erfindung der Vorrichtung zur medizinischen Früherkennung ist in der Figur 3 als Detail dargestellt und unterscheidet sich vor der ersten Ausführung durch Folgendes:
Die Kugel 4 und das Monofilament 6 sind einstückig z.B. aus Kunststoff gebildet. Hierfür ist das erste Ende des Monofilaments 6 an die Kugel 4 angeformt. Hierbei ist an dem Monofilament 6 in unmittelbarer Nähe zu der Kugel 4 oder direkt an diese angrenzend eine Sollbruchstelle ausgebildet. Diese Sollbruchstelle erlaubt ein leichtes Trennen des Monofilaments 6 von der Kugel 4.

Im Übrigen entspricht die zweite Ausführung der ersten Ausführung.

Die Benutzung der Vorrichtung ist für beide Ausführungen wie folgt:
Falls vorhanden wird die Schutzhülle entfernt.

Die Druckempfindlichkeit des Fußes - genauer einer Fußsohle - eines Diabetes- Patienten wird mittels mindestens eines der weiteren Reizgeber getestet.

Anschließend wird die Scheibe 7 um die Perforation 8 geknickt und gegebenenfalls der spitze Teil der Scheibe mit dem Oval 9b abgetrennt. Auf diese Weise wird das Monofilament 6 teilweise freigelegt und so für einen weiteren Test der Druckempfindlichkeit des Fußes vorbereitet. Für diesen weiteren Test wird die (Teil-) Scheibe 7 mit dem Monofilament 6 gefasst und dieses leicht gegen die Fußsohle gedrückt, bis eine Durchbiegung des Monofilaments 6 erkennbar ist.

Schließlich wird die (Teil-) Scheibe 7 von dem Träger 2 abgezogen und das Monofilament 6 von dem Träger 2 und aus beziehungsweise von der Kugel 4 entfernt. Der Träger 2 mit der Kugel 4 wird auf die Fußsohle geklebt, und der Patient geht damit einige Schritte als letzter Test.

Die Auswertung erfolgt wie üblich anhand der vom Patienten beschriebenen Druckempfindlichkeit.

Je nach den Gegebenheiten sind nicht alle beschriebenen Tests durchzuführen.

### Bezugszeichenliste

- 1: Pflaster
- 2: Träger
- 3: Klebstoff
- 4: Kugel
- 5: Sackbohrung
- 6: Monofilament
- 7: Scheibe
- 8: Perforation
- 9a: Grat
- 9b: Oval

## Patentansprüche

1. Vorrichtung zur medizinischen Früherkennung eines diabetischen Fußes, wobei die Vorrichtung geeignet ist, eine Druckempfindlichkeit des Fußes zu prüfen, wobei die Vorrichtung einen Klebestreifen (1) umfasst, an dem ein druckfester Körper (4) als erster Reizgeber befestigt ist, **dadurch gekennzeichnet, dass** als zweiter Reizgeber ein Monofilament (6) mit einer definierten Steifigkeit angeordnet ist, das mit einem ersten Ende in einer Sackbohrung (5) des Körpers (4) geführt ist und mit einem zweiten Ende über einen äußeren Rand des Klebestreifens (1) für eine vorbestimmte Länge herausragt.

2. Vorrichtung zur medizinischen Früherkennung eines diabetischen Fußes, wobei die Vorrichtung geeignet ist, eine Druckempfindlichkeit des Fußes zu prüfen, wobei die Vorrichtung einen Klebestreifen (1) umfasst, an dem ein druckfester Körper (4) als erster Reizgeber befestigt ist, **dadurch gekennzeichnet, dass** als zweiter Reizgeber ein Monofilament (6) mit einer definierten Steifigkeit angeordnet ist, das einteilig an den Körper (4) angeformt ist und mit einem zweiten Ende über einen äußeren Rand des Klebestreifens (1) für eine vorbestimmte Länge herausragt.

3. Vorrichtung nach Anspruch 1 oder 2, **dadurch gekennzeichnet, dass** sie eine steife Scheibe (7) umfasst, auf der der Klebestreifen (1) zusammen mit dem Körper (4) und dem Monofilament (6) befestigt ist und an deren Außenumfang mindestens ein weiterer Reizgeber (9a, 9b) geformt ist.

4. Vorrichtung nach Anspruch 3, **dadurch gekennzeichnet, dass** die Scheibe (7) aus Pappe gefertigt ist.

5. Vorrichtung nach einem der Ansprüche 1 bis 4, **dadurch gekennzeichnet, dass** der Körper in Form einer Kugel (4) einen Durchmesser von 1 mm bis 10 mm gebildet ist.

6. Vorrichtung nach einem der Ansprüche 1 bis 5, **dadurch gekennzeichnet, dass** das Monofilament (6) ab einer Kraft von 10 mN eine sichtbare Durchbiegung zeigt.

7. Vorrichtung nach Anspruch 2, **dadurch gekennzeichnet, dass** zwischen dem Körper (4) und dem Monofilament (6) eine Sollbruchstelle ausgebildet ist.

## Claims

1. Device for the medical early detection of a diabetic foot, wherein the device is suitable to check a pressure sensitivity of the foot, wherein the device comprises an adhesive strip (1), to which a pressure-resistant body (4) is fastened as a first stimulator, **characterised in that** a monofilament (6) with a defined rigidity is arranged as the second stimulator, said monofilament being guided with a first end in a blind hole (5) of the body (4) and projecting with a second end over an outer edge of the adhesive strip (1) for a predetermined length.

2. Device for the medical early detection of a diabetic foot, wherein the device is suitable to check a pressure sensitivity of the foot, wherein the device comprises an adhesive strip (1), to which a pressure-resistant body (4) is fastened as a first stimulator, **characterised in that** a monofilament (6) with a defined rigidity is arranged as the second stimulator, said monofilament (6) being formed in one piece on the body (4) and projecting with a second end over an outer edge of the adhesive strip (1) for a predetermined length.

3. Device according to claim 1 or 2, **characterised in that** it comprises a rigid disc (7), on which the adhesive strip (1) is fastened together with the body (4) and the monofilament (6) and on the outer periphery of which at least one further stimulator (9a, 9b) is formed.

4. Device according to claim 3, **characterised in that** the disc (7) is manufactured from cardboard.

5. Device according to any one of claims 1 to 4, **characterised in that** the body is in the form of a sphere (4) with a diameter of 1 mm to 10 mm.

6. Device according to any one of claims 1 to 5, **characterised in that** the monofilament (6) exhibits a visible bending from a force of 10 mN.

7. Device according to claim 2 **characterised in that** a predetermined breaking point is formed between the body (4) and the monofilament (6).

## Revendications

1. Dispositif servant à dépister de manière précoce sur le plan médical un pied diabétique, où le dispositif est adapté pour contrôler une sensibilité à la pression du pied, où le dispositif comprend une bande adhésive (1), au niveau de laquelle est fixé en tant que premier stimulateur un corps (4) résistant à la pression, **caractérisé en ce qu'**un monofilament (6) présentant une rigidité définie est disposé en tant que deuxième stimulateur, lequel est guidé, par une première extrémité, dans un alésage borgne (5) du corps (4) et dépasse, par une seconde extrémité, d'un bord extérieur de la bande adhésive (1) pour une longueur prédéterminée.

2. Dispositif servant à dépister de manière précoce sur le plan médical un pied diabétique, où le dispositif est adapté pour contrôler une sensibilité à la pression du pied, où le dispositif comprend une bande adhésive (1), au niveau de laquelle est fixé en tant que premier stimulateur un corps (4) résistant à la pression, **caractérisé en ce qu'**un monofilament (6) présentant une rigidité définie est disposé en tant que deuxième stimulateur, lequel est formé d'un seul tenant au niveau du corps (4) et dépasse, par une seconde extrémité, d'un bord extérieur de la bande adhésive (1) pour une longueur prédéterminée.

3. Dispositif selon la revendication 1 ou 2, **caractérisé en ce qu'**il comprend un disque (7) rigide, sur lequel la bande adhésive (1) est fixée conjointement avec le corps (4) et le monofilament (6) et sur la périphérie extérieure duquel est façonné au moins un autre stimulateur (9a, 9b).

4. Dispositif selon la revendication 3, **caractérisé en ce que** le disque (7) est produit à partir de carton.

5. Dispositif selon l'une quelconque des revendications 1 à 4, **caractérisé en ce que** le corps est configuré sous la forme d'une sphère (4) présentant un diamètre allant de 1 mm à 10 mm.

6. Dispositif selon l'une quelconque des revendications 1 à 5, **caractérisé en ce que** le monofilament (6) affiche, à partir d'une force de 10 mN, un fléchissement visible.

7. Dispositif selon la revendication 2, **caractérisé en ce qu'**un emplacement de rupture théorique est réalisé entre le corps (4) et le monofilament (6).
